# EUROPÄISCHE PATENTANMELDUNG

(11) **EP 0 802 190 A1**
(43) Veröffentlichungstag der Anmeldung: **22.10.1997**
(21) Anmeldenummer: 97105917.5
(22) Anmeldetag: 10.04.1997
(51) Int. Cl.: C07D 223/12, C07D 223/08, C07D 498/04

(54) **Verfahren zur Herstellung von Azepinen**

(30) Priorität: 17.04.1996 EP 96105998
(71) Anmelder: F. HOFFMANN-LA ROCHE AG, 4070 Basel (CH)
(72) Erfinder: Matzinger, Peter Karl, 4118 Rodersdorf (CH); Scalone, Michelangelo, 4127 Birsfelden (CH); Zutter, Ulrich, 4051 Basel (CH)
(74) Vertreter: Grossner, Lutz, Dr.

(57) **Zusammenfassung**

Verfahren zur Herstellung von Verbindungen der allgemeinen Formel I 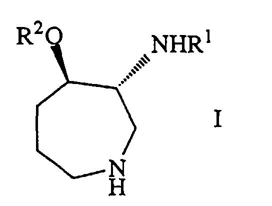 worin R¹ and R² unabhängig voneinander Aroyl bedeuten,
und neue Zwischenprodukte die in diesem Verfahren verwendet werden.

## Beschreibung

Die vorliegende Erfindung betrifft ein neues Verfahren zur Herstellung von Azepinen und in diesem Verfahren verwendete Zwischenprodukte.

In einer Hinsicht betrifft die Erfindung ein Verfahren zur Herstellung von Azepinen der allgemeinen Formel I 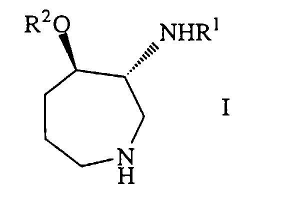 worin R¹ und R² unabhängig voneinander den Acylrest einer aromatischen Carbonsäure bedeuten.

Die vorstehende allgemeine Formel I umfasst bekannte, pharmakologisch wirksame Verbindungen, z.B. Balanol (vgl. Int. Patentanmeldung WO 93/03730) und andere Phosphokinasehemmer, wie z.B. die in der europäischen Patentanmeldung A-0 663 393 beschriebenen Verbindungen. Das erfindungsgemässe Verfahren ermöglicht es, solche Verbindungen auf einfachere und ökonomischere Weise herzustellen, als dies mit bisher bekannten Verfahren möglich war.

Erfindungsgemäss können Verbindungen der allgemeinen Formel I dadurch hergestellt werden, dass man
a) eine Verbindung der Formel II 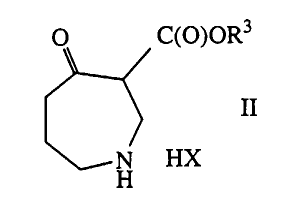 worin R³ nieder-Alkyl und HX eine Säure bedeutet,
a1) zu einer Verbindung der Formel IV 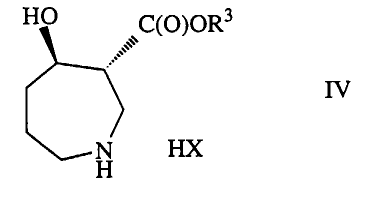 hydriert,
a2) die Verbindung der Formel IV in eine Verbindung der Formel V 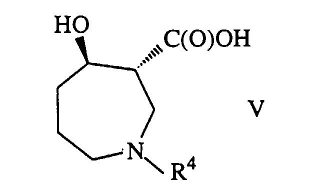 wobei R⁴ eine Schutzgruppe bedeutet,
   umwandelt,
a3) die Verbindung der Formel V in eine Verbindung der Formel VI 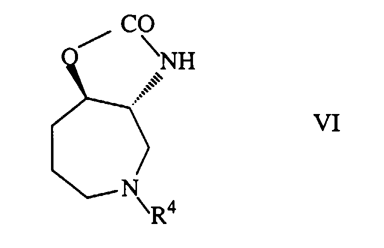 überführt,
a4) die Verbindung der Formel VI in eine Verbindung der Formel VII 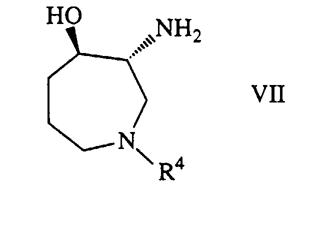 überführt,
a5) die Verbindung der Formel VII mit einer aromatischen Carbonsäure der Formel R¹COOH und/oder R²COOH N- bzw. O- acyliert;
   oder dass man
b) eine Verbindung der Formel III 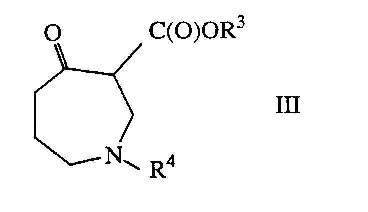 worin R³ und R⁴ die obengenannte Bedeutung haben,
b1) mikrobiell zu einer Verbindung der Formel VIII 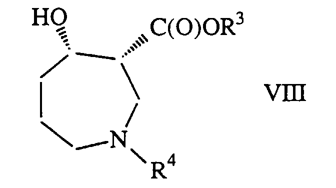 reduziert,
b2) die Verbindung der Formel VIII zu einer Verbindung der Formel VIII a verseift und diese in eine Verbindung der Formel IX 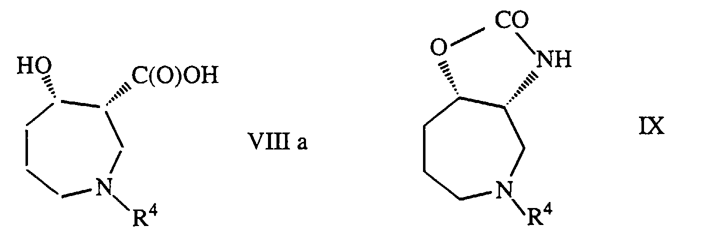 umwandelt,
b3) die Verbindung der Formel IX in eine Verbindung der Formel X 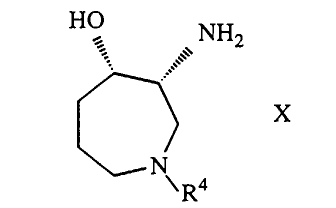 überführt,
b4) die Verbindung der Formel X mit einer aromatischen Carbonsäure der Formel R¹COOH zu einer Verbindung der Formel XI 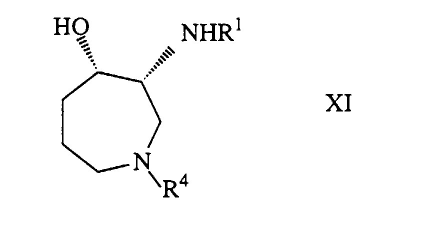 acyliert,
b5) die Verbindung der Formel XI mit einer aromatischen Carbonsäure oder einem reaktionsfähigen Derivat davon acyliert,
und aus der gemäss a5) oder b5) erhaltenen Verbindung der Formel XII 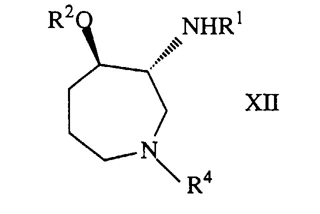 die Schutzgruppe R⁴ und gegebenenfalls in R¹ und/oder R² zusätzlich vorhandene Schutzgruppen abspaltet.

Beispiele von Acylresten R¹ und R² sind Acylreste der Benzoesäure und von substituierten, z.B. durch Hydroxy, Halogen, insbesondere Fluor, nieder-Alkyl und/oder nieder-Alkoxy, oder durch Benzoyl oder durch Fluor, nieder-Alkyl und/oder nieder-Alkoxy substituiertes Benzoyl substituierten Benzoesäuren. Der Ausdruck "nieder" bezeichnet Gruppen mit 1-6 C-Atomen. Von besonderem Interesse sind Verbindungen der Formel I, in denen R² p-Hydroxybenzoyl oder p-(2-Fluor-6-hydroxy-3-methoxybenzoyl)-benzoyl und R¹ p-Hydroxybenzoyl oder 4-Hydroxy-3,5-dimethylbenzoyl bedeuten. Als Schutzgruppen R⁴ kommen die üblicherweise verwendeten Aminoschutzgruppen in Betracht, insbesondere tert.-Butoxycarbonyl.

In einer Ausführungsform der Erfindung geht man von Verbindungen der Formel II aus, die gemäss Verfahrensstufe (a1) asymmetrisch zu einer Verbindung der Formel IV hydriert wird. Beispiele von Säuren für die Säureadditionssalze der Formel II sind anorganische Säure, wie Mineralsäuren, z.B. HCl, und organische Säuren, wie Sulfonsäuren, z.B. p-Toluolsulfonsäure und Methansulfonsäure. Diese asymmetrische Hydrierung kann mittels Komplexen von optisch aktiven, vorzugsweise atropisomeren Diphosphin-Liganden mit einem Metall der Gruppe VIII des Periodensystems insbesondere Ruthenium als Katalysator bewerkstelligt werden, die z.B. in der europäischen Patentpublikation A-0 643 052 beschrieben sind.

Beispiele solcher Katalysatoren sind

[RuL]²⁺(X)₂ III-a

[RuLX²]²⁺(X)₂ III-b

[RuLX¹X²]⁺X³ III-c

RuL(X⁴)₂ III-d

worin
- X: BF₄⁻, ClO₄⁻, B(Phenyl)₄⁻, SbF₆⁻, PF₆⁻, Z¹-SO₃⁻,
- X¹: Halogenid,
- X²: Benzol, Hexamethylbenzol oder p-Cymol,
- X³: Halogenid, ClO₄⁻, B(Phenyl)₄⁻, SbF₆⁻, PF₆⁻, Z¹-SO₃⁻ oder BF₄⁻,
- X⁴: ein Anion Z²-COO⁻ oder Z³-SO₃⁻, Allyl oder den Acetylacetonatrest (CH₃COCH=C(CH₃)O-);
- Z¹: halogeniertes niederes Alkyl oder halogeniertes Phenyl,
- Z²: niederes Alkyl, Phenyl, halogeniertes niederes Alkyl oder halogeniertes Phenyl,
- Z³: niederes Alkyl oder Phenyl bedeuten, und
- L: einen optisch aktiven, vorzugsweise atropisomeren Diphosphin-Liganden bedeuten.

Beispiele von Liganden L sind
- MeOBIPHEP: (6,6'-Dimethoxybiphenyl-2,2'-diyl)bis(diphenylphosphin)
- BIPHEMP: (6,6'-Dimethylbiphenyl-2,2'-diyl)bis(diphenylphosphin)
- BINAP: [(1,1'-Binaphthyl)-2,2'-diyl]bis(diphenylphosphin)
- pTol-BIPHEMP: (6,6'-Dimethylbiphenyl-2,2'-diyl)bis(di-(p-tolyl)phosphin)
- pAn-MeOBIPHEP: 6,6'-Dimethoxy-P,P,P',P'-tetrakis-(4-methoxyphenyl)-biphenyl-2,2'-bis-phosphin
- pDMA-MeOBIPHEP: 6,6'-Dimethoxy-P,P,P',P'-tetrakis-(4-dimethylamino-phenyl)-biphenyl-2,2'-bis-phosphin
- pPhenyl-MeOBIPHEP: (6,6'-Dimethoxybiphenyl-2,2'-diyl)bis[bis(biphenyl)-phosphin]
- mTol-BIPHEMP: (6,6'-Dimethylbiphenyl-2,2'-diyl)bis(di-(m-tolyl)phosphin)
- Cy₂-MeOBIPHEP: P2,P2-Dicyclohexyl-6,6'-dimethoxy-P2',P2'-diphenyl-biphenyl-2,2'-bis-phosphin
- 2-Furyl₂-BIPHEMP: P,P-Diphenyl-P',P'-di-2-furyl-(6,6'-dimethylbiphenyl-2,2'-diyl)diphosphin
- (3,5-Me,4-MeO)-MeOBIPHEP: 6,6'-Dimethoxy-P,P,P',P'-tetrakis-(dimethyl-4-methoxy-phenyl)-biphenyl-2,2'-bis-phosphin
- DiMeOBIPHEP: (5,5',6,6'-Tetramethoxybiphenyl-2,2'-diyl)bis(diphenylphosphin)
- TriMeOBIPHEP: (4,4',5,5',6,6'-Hexamethoxybiphenyl-2,2'-diyl)bis(diphenylphosphin) und
- 2-Furyl-MeOBIPHEP: (6,6'-Dimethoxybiphenyl-2,2'-diyl)bis(di-2-furylphosphin).

Diese Liganden sind beschrieben in den Patentpublikationen EP 643'052, EP 647'648, EP 582'692, EP 580'336, EP 690'065, EP 643'065, JP 523 9076.

Das Verhältnis von Ruthenium zu Ligand L in den Komplexen der Formeln III-a bis III-d liegt zweckmässig zwischen etwa 0,5 und etwa 2 Mol, vorzugsweise bei etwa 1 Mol, Ruthenium pro Mol Ligand. Das Substrat/Katalysator-Verhältnis (S/C; Mol/Mol) liegt zweckmässig zwischen etwa 20 und etwa 30000, vorzugsweise zwischen etwa 100 und etwa 5000.

Ein besonders bevorzugter Katalysator ist Diacetoxy-ruthenium-[(R)-6,6'-dimethoxybiphenyl-2,2'-diyl]bis(diphenylphosphin [Ru(OAc)₂(R)-MeOBIPHEP]. Zweckmässig wird die Hydrierung unter Ausschluss von Sauerstoff in Ethanol unter erhöhtem Druck, z.B. bei Drucken von 1 bis 100 bar, vorzugsweise 5-70 bar und Temperaturen von 0 bis 80°C, vorzugsweise 20-50°C, durchgeführt. In der Verfahrensstufe (a2) wird in der so erhaltenen Verbindung der Formel IV die NH-Gruppe, z.B. durch eine tert.-Butoxycarbonylgruppe, geschützt. Nach Verseifung der Estergruppe R³, die zweckmässig mit wässrigem Alkali, z.B. Natronlauge bei Raumtemperatur, vorgenommen wird, wird die nach Ansäuern erhaltene Carbonsäure der Formel V in das Oxazolidon der Formel VI überführt (a3). Diese Reaktion kann in an sich bekannter Weise durch Ueberführung der Carboxygruppe in der Verbindung der Formel V in ein Säureazid oder Säureamid der Formel Va 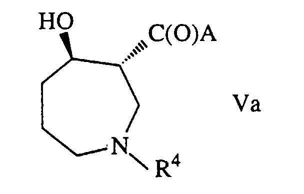 wobei A Azido oder Amino bedeutet,
und anschliessendem Abbau nach Curtius oder Hofmann durchgeführt werden. Das Oxazolidon VI kann in an sich bekannter Weise, z.B. mit wässrig-alkoholischem Alkali, zweckmässig unter Erwärmen auf 70-90 °C, zur Verbindung VII hydrolysiert werden (a4). In Verfahrensstufe (a5) können die Hydroxy- und die Aminogruppe in der Verbindung der Formel VII in an sich bekannter Weise acyliert werden, z.B. durch Umsetzung mit einem reaktionsfähigem Derivat einer Carbonsäure R¹COOH bzw. R²COOH, wie einem gemischtem Anhydrid. Sofern diese Carbonsäuren acylierbare Gruppen, wie OH-Gruppen enthalten, werden solche Gruppen zweckmässigerweise intermediär geschützt. Verbindungen der Formel I, in denen R¹ und R² voneinander verschieden sind, können z.B. dadurch erhalten werden, dass man die Aminogruppe der Verbindung der Formel VII selektiv mit 1 Aequivalent R¹COOH N-acyliert und anschliessend mit 1 Aequivalent R²COOH O-acyliert.

In einer anderen Ausführungsform des erfindungsgemässen Verfahrens geht man von Verbindungen der allgemeinen Formel III aus, die in Verfahrensstufe (b1) mikrobiell zu Verbindungen der Formel VIII reduziert werden können. Die Reduktion ist grundsätzlich nicht an einen spezifischen Mikroorganismus gebunden. Zweckmässig verwendet man als Mikroorganismen Pilzstämme (Fungi), insbesondere Hefen. Ein bevorzugter Mikroorganismus ist Hanseniaspora uvarum R 1052, insbesondere der am 16.1.1996 bei der Deutschen Sammlung von Mikroorganismen und Zellkulturen (DSMZ) unter der Nr. DSM 10 496 hinterlegte Stamm. Die Reduktion einer Verbindung III zu einer Verbindung der Formel IV kann mittels intakter Zellkulturen oder mittels aus den Mikroorganismen gewonnener Enzyme vorgenommen werden. Der bevorzugte Mikroorganismus, Hanseniaspora uvarum R 1052 kann auf üblichen Nährböden, die Kohlenstoff- und Stickstoffquellen z.B. Glucose oder Stärke, bzw. Sojamehl, Hefeextrakt oder Pepton, sowie anorganische Salze, wie Ammoniumsulfat, Natriumchlorid oder Natriumnitrat enthalten, in aeroben wässrigen Submerskulturen kultiviert werden. Die Kultivierung kann bei Temperaturen von etwa 20-35 °C, vorzugsweise bei 27 °C, in einem pH-Bereich von etwa 3-9, vorzugsweise bei etwa pH 5-7 durchgeführt werden. Die Verbindung der Formel III wird der Kultur des Mikroorganismus zweckmässig in einem organischen Lösungsmittel, z.B. Aethylacetat zugesetzt. Der Fortschritt der Reduktion kann durch Dünnschichtchromatographie von Proben des Reaktionsmediums verfolgt werden. Im allgemeinen beträgt die Reaktionsdauer etwa 8-12 Stunden. Das Reaktionsprodukt, die Verbindung der Formel IV, kann aus der Kulturlösung durch Extraktion mit einem geeigneten organischen Lösungsmittel, z.B. mit Aethylacetat, abgetrennt werden. In der nächsten Reaktionsstufe (b2) wird die Verbindung der Formel VIII zur entsprechenden Carbonsäure verseift und diese in ein Oxazolidon der Formel IX übergeführt. Die Ueberführung der aus der Verbindung VIII erhaltenen Carbonsäure in das Oxazolidon IX kann in Analogie zu den bei (a3) beschriebenen Verfahren durchgeführt werden. Durch alkalische Hydrolyse des Oxazolidons IX (b3) in Analogie zu dem bei (a4) beschriebenen Verfahren erhält man die Verbindung der allgemeinen Formel X, die in Analogie zu dem bei (a5) beschriebenen Verfahren zu einer Verbindung der allgemeinen Formel XI N-acyliert wird (Verfahrensstufe (b4)). Eine Verbindung der allgemeinen Formel XI kann durch Acylierung mit einer Carbonsäure der allgemeinen Formel R²OH in Gegenwart von Triphenylphosphin und Diethyl-azo-dicarboxylat in eine Verbindung der allgemeinen Formel XII umgewandelt werden (Verfahrensstufe (b5)). Aus der gemäss (a5) oder (b5) erhaltenen Verbindung der allgemeinen Formel XII kann die Schutzgruppe R⁴ in an sich bekannter Weise entfernt werden, im Fall einer tert.-Butoxycarbonylgruppe z.B. durch Behandlung mit einer Säure, wie 2N HCl in einem Lösungsmittel wie Aethylazetat.

Die in dem erfindungsgemässen Verfahren auftretenden Verbindungen der allgemeinen Formeln II, IV, V, VI, VIII, VIIIa, IX, X und XI sowie die in Beispiel 7a bzw. 12 hergestellte Verbindung sind neu und ebenfalls Gegenstand der Erfindung.

Die Erfindung wird durch die nachstehenden Beispiele weiter erläutert, wobei Beispiel 1 die Herstellung der als Ausgangsmaterial eingesetzten Verbindungen der Formel II und III beschreibt.

### Beispiel 1

a) Zu 101,2 g Piperidin-3-ol in 750 ml Dichlormethan wurde eine Lösung von 218,3 g di-tert-Butyldicarbonat in 250 ml Dichlormethan bei 20-25°C unter Rühren im Verlauf von 1 Stunde gegeben. Das Reaktionsgemisch wurde weitere 2 Stunden bei Raumtemperatur gerührt. Danach wurde eine Lösung von 33,6 g Natriumbicarbonat und 11,9 g Kaliumbromid in 1000 ml entionisiertem Wasser zugesetzt und das Reaktionsgemisch auf -2°C gekühlt. Nach Zusatz von 0,39 g 2,2,6,6-Tetramethyl-piperidin-1-oxid wurden 560 g 13,3%ige wässrige Natriumhypochloritlösung im Verlauf von 80 Minuten bei 0-5°C zugegeben. Nach weiteren 30 Minuten Rühren bei -2°C wurde der Ueberschuss von Natriumhypochlorit durch Zusatz von etwa 10 ml 38%iger wässriger Natriumbisulfitlösung zerstört. Das Reaktionsgemisch wurde dann auf 20°C aufgewärmt, die wässrige Schicht abgetrennt und mit 500 ml Dichlormethan extrahiert. Beide organische Phasen wurden mit 500 ml 10%iger Kochsalzlösung gewaschen, vereinigt und über Natriumsulfat getrocknet. Nach Filtration und Entfernen des Lösungsmittels unter vermindertem Druck wurde der ölige Rückstand durch Destillation unter vermindertem Druck gereinigt. Man erhielt 191,2 g 3-Oxo-piperidin-1-carbonsäure-tert-butylester als farbloses Oel, Siedepunkt 80-82°C/0,01 mbar.
b) 99,6 g der in Absatz a) erhaltenen Verbindung wurden in 600 ml Diethylether gelöst. Die Lösung wurde auf -70°C gekühlt und die weisse Suspension gleichzeitig und tropfenweise im Verlauf von 1 Stunde mit Lösungen von 62,0 ml Ethyldiazoacetat in 125 ml Diethylether und 69,0 ml Bortrifluoridetherat in 125 ml Diethylether versetzt, wobei die innere Temperatur bei -70°C gehalten wurde. Nach weiterem Rühren bei -70°C während 1 Stunde wurde das Kühlbad entfernt, das Reaktionsgemisch auf 0°C aufgewärmt und mit 375 ml 10%iger Natriumcarbonatlösung versetzt. Die wässrige Phase wurde abgetrennt und mit 250 ml Diethylether extrahiert. Die organischen Phasen wurden mit 250 ml 10%iger Kochsalzlösung gewaschen, vereinigt und über Natriumsulfat getrocknet. Nach Entfernen des Lösungsmittels unter vermindertem Druck bei 30°C wurde 1-(tert-Butoxycarbonyl)-4-oxo-azepan-3-carbonsäure-ethylester als Rohprodukt in Form eines gelben Oels erhalten, das ohne weitere Reinigung im nächsten Schritt eingesetzt wurde.
c) 147,2 g des in Absatz b) erhaltenen Produktes wurden in 1250 ml Dioxan gelöst und mit 0,1 g 4-Oxo-azepan-3-carbonsäure-ethylester-hydrobromid beimpft. Danach wurden bei Raumtemperatur unter Rühren im Verlauf von 25 Minuten 175 ml 5,7M HBr/Essigsäure zugesetzt. Nach weiterem Animpfen mit 0,1 g 4-Oxo-azepan-3-carbonsäure-ethylester-hydrobromid wurde die Suspension 5 Stunden bei Raumtemperatur gerührt. Die Kristalle wurden abfiltriert und mit Ethylacetat gewaschen und bei 50°C und 25 mbar getrocknet. Man erhielt 91,0 g rohes 4-Oxo-azepan-3-carbonsäure-ethylesterhydrobromid, das unter Rühren und Rückflusserhitzen in 1250 ml 2-Butanon gelöst wurde. Die Lösung wurde auf 65°C gekühlt und mit 0,1 g reinem 4-Oxo-azepan-3-carbonsäure-ethylester-hydrobromid angeimpft. Nach Abkühlen auf Raumtemperatur wurde die Suspension 1 Stunde bei Raumtemperatur und 3 Stunden bei 0°C gerührt. Die Kristalle wurden abfiltriert, mit 200 ml auf -10°C gekühltem 2-Butanon gewaschen und bei 50°C und 25 mbar getrocknet. Man erhielt 68,2 g weisses 4-Oxo-azepan-3-carbonsäure-ethylester-hydrobromid vom Schmelzpunkt 127-130°C (Zers.).
d) 59,4 g der in Absatz b) erhaltenen Verbindung wurden in 1000 ml 1M HCl in Dioxan gelöst und 24 Stunden bei Raumtemperatur gerührt. Nach 1,5 Stunden Reaktionsdauer wurde die Lösung mit ca. 25 mg 4-Oxo-azepan-3-carbonsäure-ethylester-hydrochlorid beimpft. Die weisse Suspension wurde abfiltriert, mit Dioxan gewaschen und bei 50°C und 25 mbar getrocknet. Man erhielt 31,3 g 4-Oxo-azepan-3-carbonsäure-ethylester-hydro chlorid in Form weisser Kristalle, die gemäss NMR-Spektrum etwa 0,4 Mol Dioxan pro Mol Hydrochlorid enthielten. Zur weiteren Reinigung und Entfernung des Dioxans wurde das Hydrochlorid umkristallisiert. 31,3 g 4-Oxo-azepan-3-carbonsäure-ethylester-hydrochlorid wurden in 600 ml 2-Butanol bei 80°C gelöst, die Lösung auf -20°C im Verlauf von 2 Stunden abgekühlt und 3 Stunden bei -20°C gerührt. Die weisse Suspension wurde abfiltriert, mit 2-Butanol (auf-20°C gekühlt) gewaschen und bei 50°C und 25 mbar getrocknet. Man erhielt 22,9 g 4-Oxo-azepan-3-carbonsäure-ethylester-hydrochlorid in Form weisser Kristalle, Schmelzpunkt 145-148°C (Zers.).

### Beispiel 2

75,0 g 4-Oxo-azepan-3-carbonsäure-ethylester-hydrobromid und 800 ml Ethanol wurden in einen Autoklaven eingebracht. Der Autoklav wurde verschlossen und die Luft daraus durch wiederholtes Evakuieren auf ca. 0,1 bar und Aufdrücken von Argon (7 bar) und Wasserstoff (40 bar) unter Rühren entfernt. Danach wurden bei 2 bar Wasserstoffdruck unter Sauerstoffausschluss eine Lösung von 226 mg Diacetoxy-rhuthenium(R)-6,6'-dimethoxybiphenyl-2,2-diyl)-bis(diphenylphosphin) in 20 ml Ethanol in den Autoklaven eingespeist. Danach wurden 40 bar Wasserstoff aufgedrückt und das Reaktionsgemisch unter Rühren 19 Stunden bei 30°C und 3 Stunden bei 50°C hydriert. Danach wurde der Inhalt des Autoklaven mit 200 ml Ethanol ausgewaschen und die vereinigten Lösungen bei 50°C/100 mbar eingedampft und der braune Rückstand während 2 Stunden getrocknet. Der Rückstand (75,9 g, bestehend aus ca. 80% 3R,4R und 20% 3S,4R-Isomeren) wurde mit 450 ml Tetrahydrofuran während 19 Stunden bei 24°C und 1 Stunde bei 16°C verrührt. Die Kristalle wurden abgenutscht, mit Tetrahydrofuran gewaschen und 3,5 Stunden bei 50°C/20 mbar bis zur Gewichtskonstanz getrocknet. Man erhielt 56,3 g leicht beige Kristalle, die erneut mit 225 ml Tetrahydrofuran, wie vorstehend beschrieben, verrührt wurden. Nach Abnutschen und Trocknen der Kristalle wurden 55,1 g (3R,4R)-4-Hydroxy-azepan-3-carbonsäure-ethylester-hydrobromid in Form weisser Kristalle erhalten, die nach HPLC enantiomerenrein waren.

### Beispiel 3

Wie in Beispiel 2 wurden 23,2 g 4-Oxo-azepan-3-carbonsäure-ethylesterhydrochlorid in 90 ml Ethanol mit einer Lösung von 36,1 mg des Rutheniumkatalysators in 10 ml Ethanol unter 40 bar Wasserstoffdruck während 21 Stunden bei 30°C und während 3 Stunden bei 50°C hydriert. Der Rückstand, bestehend aus ca. 80% 3R,4R und 20% 3S,4R-Isomeren, wurde mit Tetrahydrofuran und Ethanol eine halbe Stunde bei 50°C und 4 Stunden bei Raumtemperatur verrührt, die Kristalle abgenutscht, mit wenig Tetrahydrofuran/Ethanol gewaschen und bis zur Gewichtskonstanz bei 50°C/20 mbar getrocknet. Man erhielt 13,3 g enantiomerenreines (3R,4R)-4-Hydroxyazepan-3-carbonsäure-ethylester-hydrochlorid in Form weisser Kristalle.

### Beispiel 4

Wie in Beispiel 2 wurden 0.44 g 4-Oxo-azepan-3-carbonsäure-ethylester hydrochlorid in 9 ml Ethanol mit einer Lösung von 3.2 mg Di(η²-acetato)(η⁴-cycloocta-1,5-dien)ruthenium(II) und 5.8 mg (R)-MeOBIPHEP in 1 ml Diethylether/THF 3/1 unter 40 bar Wasserstoffdruck während 23.5 Stunden bei 25°C hydriert. Die gelbe Hydrierlösung wurde am Rotationsverdampfer bei 40°/20 mbar eingedampft. Bei einem Umsatz von 83% bestand der Rückstand gemäss HPLC-Analyse aus 65% (3R, 4R)-4-Hydroxy-azepan-3-carbonsäure-ethylester hydrochlorid mit einem ee >99%.

### Beispiel 5

In zu den Beispielen 2-4 analoger Weise wurden die in Tabelle 1 aufgeführten Hydrierungen durchgeführt.

**Tabelle 1**

| Asymmetrische Hydrierung von 4-Oxo-azepan-3-carbonsäure-ethylester.HX¹⁾ | | | | | | | | | | |
|---|---|---|---|---|---|---|---|---|---|---|
| Bsp Nr | L | X | Lsm | T °C | Druckbar | Ums./h | trans³⁾ | | cis | |
| | | | | | | | % | ee | % | ee |
| 5a | (S)-BINAP | Cl | 2) | 25 | 40 | 62/23 | 78 | >99 | 22 | 73 |
| 5b | (R)-BIPHEMP | Cl | 2) | 25 | 40 | 90/23 | 66 | 94 | 34 | 38 |
| 5c | (R)-pTol-BIPHEMP | Cl | 2) | 25 | 40 | 93/23 | 72 | >99 | 28 | 62 |
| 5d | (R)-p-An-MeOBIPHEP | Cl | 2) | 25 | 40 | 87/23 | 80 | >99 | 20 | 77 |
| 5e | (R)-mTol-BIPHEMP | Cl | 2) | 25 | 40 | 90/24 | 58 | 97 | 42 | 47 |
| 5f | (R)-pDMA-MeOBIPHEP | Cl | 2) | 25 | 40 | 79/24 | 72 | >99 | 28 | 95 |
| 5g | (R)-pPhenyl-MeOBIPHEP | Cl | 2) | 25 | 40 | 54/23 | 82 | >99 | 18 | 26 |
| 5h | (S)-3,5-Me,4-MeO-MeOBIPHEP | Cl | 2) | 25 | 40 | 34/23 | 55 | >99 | 45 | 61 |
| 5i | (R)-DiMeOBIPHEP | Cl | 2) | 25 | 40 | 99/23 | 66 | >99 | 34 | 86 |
| 5j | (R)-MeOBIPHEP | Br | EtOH | 40 | 100 | 99/21 | 76 | >99 | 24 | 84 |
| 5k | (R)-MeOBIPHEP | Br | EtOH | 60 | 100 | 100/21 | 69 | >99 | 31 | 85 |
| 5l | (R)-2-Furyl-MeOBIPHEP | Br | EtOH | 40 | 100 | 76/29 | 64 | 98 | 36 | 95 |
| 5m | (R)-2-Furyl-2-Biphemp | Br | EtOH | 40 | 100 | 94/21 | 68 | >99 | 32 | 69 |
| 5n | (R)-TriMeOBIPHEP | Br | EtOH | 30 | 100 | 100/23 | 76 | >99 | 24 | 95 |
| 5o | (R)-Cy2-MeOBIPHEP | Cl | EtOH | 80 | 20 | 100/22 | 38 | >99 | 62 | 92 |
| 5p | (R)-MeOBIPHEP | Cl | MeOH | 30 | 100 | 100/22 | 75 | >99 | 25 | 88 |
| 5q | (R)-MeOBIPHEP | Cl | iPrOH | 30 | 100 | 90/22 | 78 | >99 | 22 | 84 |
| 5r | (R)-MeOBIPHEP | Cl | AcOH | 25 | 40 | 97/23 | 5 | >99 | 95 | 94 |

| | | | | | | | | | | |
|---|---|---|---|---|---|---|---|---|---|---|
| 1) Katalysatorherstellung analog Beispiel 2 und 3. | | | | | | | | | | |
| 2) Katalysatorherstellung: in situ analog Beispiel 4, Lösungsmittel: Ethanol-Diethylether-Tetrahydrofuran 9:0.65:0.35. | | | | | | | | | | |
| 3) trans: Verbindung IV oder sein Enantiomer. Chirale Diphosphinliganden mit (R)-Konfiguration ergeben (3R,4R)-IV . | | | | | | | | | | |

### Beispiel 6

Wie in Beispiel 3 wurden 3.32 g 4-Oxo-azepan-3-carbonsäure-ethylester hydrochlorid in 38 ml Ethanol in Anwesenheit von 6.3 mg [RuCl((R)-MeOBIPHEP)(C6H6)]Cl unter 40 bar Wasserstoffdruck während 19 Stunden bei 30°C und während 3 Stunden bei 50° hydriert. Die gelbe Hydrierlösung wurde am Rotationsverdampfer bei 40°/20 mbar eingedampft. Bei einem Umsatz von 95% bestand der Rückstand gemäss HPLC-Analyse aus 79% (3R, 4R)-4-Hydroxy-azepan-3-carbonsäure-ethylester hydrochlorid mit einem ee >99%.

### Beispiel 7

In einer Glove Box (O₂-Gehalt < 1ppm) wurde eine Katalysatorlösung durch Lösen von 1.3 ml einer 0.03 molaren ethanolischen HBr-Lösung und von 16.1 mg Ru(OAc)2((R)-MeOBIPHEP) in 10 ml Ethanol hergestellt und 0.5 Stunden gerührt. Dann wurden 0.53g 4-Oxo-azepan-3-carbonsäureethylester hydrobromid und 2 ml der oben hergestellte Katalysatorlösung in 4 ml Ethanol in einem Autoklaven vorgelegt und unter 100 bar Wasserstoffdruck während 21 Stunden bei 20°C hydriert. Die gelbe Hydrierlösung wurde am Rotationsverdampfer bei 40°/20 mbar eingedampft. Bei einem Umsatz von 76% bestand der Rückstand gemäss HPLC-Analyse aus 58% (3R, 4R)-4-Hydroxy-azepan-3-carbonsäure-ethylester hydrobromid mit einem ee >99%.

### Beispiel 8

In einer Glove Box (O₂-Gehalt < 1ppm) wurde eine Katalysatorlösung durch Lösen von 1.0 ml einer 0.04 molaren ethanolischer HBF4-Lösung und von 32.1 mg Ru(OAc)2((R)-MeOBIPHEP) in 10 ml Ethanol hergestellt und 0.5 Stunden gerührt. Dann wurden 0.53g 4-Oxo-azepan-3-carbonsäureethylester hydrobromid und 1 ml der oben hergestellte Katalysatorlösung in 9 ml Ethanol in einem Autoklaven vorgelegt und unter 100 bar Wasserstoffdruck während 21 Stunden bei 20°C hydriert. Die gelbe Hydrierlösung wurde am Rotationsverdampfer bei 40°/20 mbar eingedampft. Bei einem Umsatz von 44% bestand der Rückstand gemäss HPLC-Analyse aus 37% (3R,4R)-4-Hydroxy-azepan-3-carbonsäure-ethylester hydrobromid mit einem ee >99%.

### Beispiel 9

67,0 g (3R,4)-4-Hydroxy-azepan-3-carbonsäure-ethylester-hydrobromid wurden in 500 ml tert-Butyl-methylether suspendiert und mit 30,4 g Triethylamin versetzt. Danach wurden eine Lösung von 54,6 g di-tert-Butyl-dicarbonat in 25 ml tert-Butyl-methylether bei Raumtemperatur im Verlauf von 20 Minuten zugesetzt. Danach wurde bei Raumtemperatur weitere 2 Stunden gerührt.

Zu der weissen Suspension wurden 500 ml 2N NaOH gegeben und das Reaktionsgemisch wurde 2 Stunden kräftig bei Raumtemperatur gerührt. Das Reaktionsgemisch wurde dann mit 175 ml 6N HCl angesäuert und nach Phasentrennung wurde die. wässrige Phase zweimal mit 100 ml tert-Butyl-methylether extrahiert. Alle organischen Phasen wurden mit 150 ml 10%iger Kochsalzlösung gewaschen, vereinigt und über Natriumsulfat getrocknet. Nach Entfernen des Lösungsmittels unter vermindertem Druck bei 40°C wurde die rohe Hydroxysäure in 260 ml Butylacetat bei etwa 85°C gelöst. Nach Animpfen mit reinem Endprodukt wurde die Suspension auf -20°C im Verlauf von 2 Stunden gekühlt und über Nacht bei dieser Temperatur gerührt. Die Suspension wurde abfiltriert, mit 100 ml Hexan gewaschen und bei 50°C und 25 mbar getrocknet. Man erhielt 55,9 g (3R,4R)-4-Hydroxy-azepan-1,3-dicarbonsäure-1-tert-butylester, Schmelzpunkt 121,5-122,5°C.

### Beispiel 10

Zu 38,9 g der in Beispiel 9 hergestellten Verbindung wurden 300 ml Ethylacetat und 20,9 ml Triethylamin gegeben. Die Lösung wurde zum Rückflusssieden erhitzt, sodann wurden im Verlauf von 30 Minuten 32,4 ml Diphenylphosphorylazid zugegeben und das Rückflusserhitzen weitere 2 Stunden fortgesetzt. Nach Abkühlen auf Raumtemperatur wurde das Reaktionsgemisch mit 300 ml Ethylacetat versetzt, mit 150 ml 5%iger Natriumhydrogencarbonatlösung und zweimal mit 150 ml Wasser gewaschen. Die wässrigen Phasen wurden zweimal mit 300 ml Ethylacetat extrahiert. Die vereinigten organischen Phasen wurden über Natriumsulfat getrocknet und unter vermindertem Druck bei 45°C eingedampft. Der rohe kristalline Rückstand wurde in 300 ml Butylacetat gelöst, mit reinem Endprodukt angeimpft, auf -20°C im Verlauf von ca. 3 Stunden abgekühlt und über Nacht gerührt. Die Suspension wurde filtriert, auf -20°C vorgekühltem Butylacetat gewaschen, bei 60°C und 25 mbar getrocknet und lieferte 29,9 g (3aR,8aR)-5-tert-Butoxycarbonyl-2-oxo-octahydro-oxazolo-[4,b-c]azepin vom Schmelzpunkt 152,5-153,5°C.

### Beispiel 11

25,6 g der in Beispiel 10 hergestellten Verbindung wurden zu 250 ml Methanol und 250 ml 2N NaOH gegeben. Das Reaktionsgemisch wurde zum Rückfluss erhitzt und 3 Stunden bei dieser Temperatur gehalten. Nach Abkühlen wurden 265 ml Lösungsmittel bei 50°C und 150 mbar abdestilliert und der Rückstand dreimal mit je 200 ml Ethylacetat extrahiert. Die drei organischen Phasen wurden mit 50 ml 10%iger Kochsalzlösung gewaschen, vereinigt und über Natriumsulfat getrocknet. Nach Entfernen des Lösungsmittels wurde das als Rückstand erhaltene viskose Oel in 100 ml Cyclohexan bei 60°C gelöst, mit reinem Endprodukt beimpft, im Verlauf von 2 Stunden auf Raumtemperatur abgekühlt und über Nacht gerührt. Die Suspension wurde filtriert, mit 40 ml Cyclohexan gewaschen und bei 50°C und 25 mbar getrocknet, wobei 21,5 g (3R,4R)-3-Amino-4-hydroxy-azepan-1-carbonsäure-tert-butylester, Schmelzpunkt 99-100,5°C, erhalten wurden.

### Beispiel 12

a) Zu 4,66 g 4-tert-Butoxybenzoesäure und 6,11 g 4-Dimethylaminopyridin in 30 ml Dichlormethan wurden bei Raumtemperatur 4,58 g p-Toluolsulfonylchlorid, gelöst in 24 ml Dichlormethan, im Verlauf von 10 Minuten gegeben. Nach 2-stündigem Rühren bei Raumtemperatur wurden 2,30 g der in Beispiel 6 hergestellten Verbindung in 6 ml Dichlormethan im Verlauf von 10 Minuten zugegeben. Danach wurde 16 Stunden bei Raumtemperatur gerührt. Das Reaktionsgemisch wurde zweimal mit je 20 ml 1N NaOH gewaschen, sodann mit 40 ml 1N HCl und 40 ml Wasser. Alle wässrigen Phasen wurden mit 20 ml Dichlormethan extrahiert. Die vereinigten organischen Phasen wurden über Natriumsulfat getrocknet und das Lösungsmittel wurde unter vermindertem Druck entfernt. Der zurückbleibende weisse Schaum wurde über 300 g Silicagel mit 6,5 l HexanEthylacetat (2:1) chromatographiert. Es wurden 250 ml-Fraktionen gesammelt. Die Fraktionen 8-25 wurden vereinigt und das Lösungsmittel unter vermindertem Druck abgedampft, wobei 5,91 g weisser Schaum erhalten wurden, der in 80 ml Heptan bei 60°C gelöst wurde. Nach Rühren bei -20°C über Nacht wurden die Kristalle abfiltriert, mit kaltem Heptan gewaschen und bei 50°C und 25 mbar getrocknet. Man erhielt 5,34 g (3R,4R)-3-(4-tert-Butoxy-benzoylamino)-4-(4-tert-butoxy-benzoyloxy)-azepan-1-carbonsäure-tert-butylester vom Schmelzpunkt 125,5-127,5°C.
b) Zu 5,83 g der in Abschnitt a) erhaltenen Verbindung, in 30 ml Ethylacetat gelöst, wurden bei Raumtemperatur und unter Rühren 20,0 ml 5M HCl in Ethylacetat gegeben. Das Reaktionsgemisch wurde über Nacht bei Raumtemperatur gerührt, der weisse Niederschlag abfiltriert und dreimal mit je 5 ml Ethylacetat gewaschen und 16 Stunden bei 50°C/25 mbar getrocknet. Das erhaltene weisse Pulver wurde in 50 ml Wasser gelöst und 1 Stunde bei 50°C gerührt. Die Lösung wurde dann lyophilisiert und lieferte 3,97 g reines 3-(4-Hydroxy-benzoylamino)-4-(4-hydroxy-benzoyloxy)-hexahydroazepin-hydrochlorid.

### Beispiel 13

Hanseniaspora uvarum R 1052 wurde 3 Tage bei 27°C in einer Petrischale mit festem Nährboden angezüchtet. Nach 3 Tagen wurden 100 ml flüssiges Nährmedium in einer 500 ml-Schüttelflasche mit einer Oese dieser Kultur beimpft. Diese Vorkultur wurde 18 Stunden bei 27°C geschüttelt. Die Zellen wuchsen zu einer Dichte von 5 x 10⁸ Zellen/ml (stationäre Phase). Die gesamte Vorkultur wurde zur Beimpfung eines Reaktors benutzt, der 7500 ml Nährmedium (enthaltend 1% Hefeextrakt Difco: Bacto Yeast Extract # 0127-17-9, 1% Pepton Difco: Bacto Peptone # 0118-17-0 und 2% Glukose in entionisiertem Wasser). Nach 18 Stunden wurden 750 ml 50%ige Glukoselösung und unmittelbar darauf 29 g der in Beispiel 1b hergestellten Verbindung, gelöst in 20 ml Ethylacetat im Verlauf von 25 Minuten zugegeben. Nach 12 Stunden wurde die Kulturlösung zweimal mit je 2000 ml Ethylacetat extrahiert. Die vereinigten organischen Phasen wurden über Natriumsulfat getrocknet. Nach Entfernung des Lösungsmittels unter vermindertem Druck bei 30°C wurde 30,1 g (3R,4S)-1-(tert-Butoxycarbonyl)-4-hydroxy-azepan-3-carbonsäure-ethylester als viskoses oranges Oel erhalten.

### Beispiel 14

a) Ein Gemisch von 28,7 g der in Beispiel 13 hergestellten Verbindung in 200 ml tert-Butylmethylether und 200 ml 2N NaOH wurde 4 Stunden kräftig bei Raumtemperatur gerührt und dann 20 Stunden bei 50°C. Nach Abkühlen wurde die wässrige Phase zweimal mit je 100 ml tert-Butylmethylether extrahiert. Die organischen Phasen wurden verworfen. Die wässrige Phase wurde vorsichtig mit ca. 70 ml 6N HCl angesäuert, einmal mit 200 ml und zweimal mit je 100 ml tert-Butylmethylether extrahiert. Alle drei organischen Phasen wurden einzeln mit 50 ml 10%iger Kochsalzlösung gewaschen, vereinigt und über Natriumsulfat getrocknet. Nach Entfernung des Lösungsmittels unter vermindertem Druck (40°C/25 mbar) wurde das braune viskose Oel in 60 ml Isopropylether bei 60°C gelöst und 16 Stunden bei -20°C kristallisieren gelassen. Die Kristalle wurden abfiltriert, mit wenig auf -20°C gekühltem Isopropylether gewaschen und 5 Stunden bei 40°C und 25 mbar getrocknet. Man erhielt 12,0 g (3R,4S)-4-Hydroxy-azepan-1,3-dicarbonsäure-1-tert-butylester vom Schmelzpunkt 98,5-101,5°C.
b) Zu 18,1 g der in Absatz a) erhaltenen Verbindung wurden 140 ml Ethylacetat, 9,8 ml Triethylamin und 15,9 ml Diphenylphosphorylazid gegeben. Die Lösung wurde 2 Stunden zum Rückfluss erhitzt, das Reaktionsgemisch abgekühlt, mit 140 ml Ethylacetat verdünnt und mit 70 ml 5%iger Natriumhydrogencarbonatlösung und zweimal mit je 70 ml Wasser gewaschen. Die drei wässrigen Phasen wurden getrennt, dreimal mit 140 ml Ethylacetat gewaschen. Die vereinigten organischen Phasen wurden über Natriumsulfat getrocknet und das Lösungsmittel bei 45°C/25 mbar entfernt. Der rohe kristalline Rückstand wurde in 140 ml Butylacetat bei ca. 80°C gelöst, mit reinem Endprodukt beimpft, abgekühlt und 6 Stunden bei -20°C gerührt. Die Suspension wurde filtriert, mit auf -20°C gekühltem Butylacetat gewaschen, bei 60°C und 25 mbar über Nacht getrocknet, wobei 13,3 g (3aR,8aS)-2-Oxo-octahydro-oxazolo[4,b-c]azepin-5-carbonsäure-tert-butylester vom Schmelzpunkt 158-159°C erhalten wurden.

### Beispiel 15

Zu 20,5 g der in Beispiel 14b) hergestellten Verbindung wurden 200 ml Methanol und 200 ml 2N NaOH gegeben. Das Reaktionsgemisch wurde zum Rückfluss erhitzt und 4 Stunden bei dieser Temperatur belassen. Nach Abkühlen wurden 200 ml Methanol bei 50°C und 150 mbar abdestilliert und der Rückstand dreimal mit je 160 ml Ethylacetat extrahiert. Die organischen Phasen wurden mit 40 ml 10%iger Kochsalzlösung gewaschen, vereinigt und über Natriumsulfat getrocknet. Nach Entfernung des Lösungsmittels wurde das als Rückstand erhaltene viskose Oel in 80 ml Methylcyclohexan bei 50°C gelöst, mit reinem Endprodukt beimpft, abgekühlt und 4 Stunden bei 0°C gerührt. Die Kristalle wurden abfiltriert, mit 20 ml Methylcyclohexan gewaschen und bei 50°C und 25 mbar über Nacht getrocknet. Man erhielt 17,4 g (3R,4S)-3-Amino-4-hydroxy-azepan-1-carbonsäure-tert-butylester, Schmelzpunkt 64-67°C.

### Beispiel 16

Zu 11,5 g 4-(tert-Butoxy)-benzoesäure und 13,1 g 4-Dimethylaminopyridin in 100 ml Dichlormethan wurden bei Raumtemperatur 9,06 g p-Toluolsulfonylchlorid in 75 ml Dichlormethan gegeben. Das Reaktionsgemisch wurde weitere 2 Stunden gerührt. Die Lösung wurde dann im Verlauf von 1 Stunde zu 11,5 g der in Beispiel 10 hergestellten Verbindung, gelöst in 50 ml Dichlormethan, gegeben. Nach Rühren während 1 Stunde bei Raumtemperatur wurde das Reaktionsgemisch mit 100 ml 1N NaOH, 100 ml 1N HCl und 100 ml Wasser gewaschen. Alle wässrigen Phasen wurden mit 50 ml Dichlormethan extrahiert. Die vereinigten organischen Phasen wurden über Natriumsulfat getrocknet und das Lösungsmittel unter vermindertem Druck abgetrennt. Der schaumige Rückstand wurde in 400 ml heissem Heptan gelöst und bei Raumtemperatur über Nacht kristallisieren gelassen. Die Kristalle wurden mit 25 ml Heptan gewaschen und bei 50°/25 mbar getrocknet. Man erhielt 17,3 g (3R,4S)-3-(4-tert-Butoxybenzoylamino)-4-hydroxy-azepan-1-carbonsäure-tert-butylester vom Schmelzpunkt 131,5-132,5°C.

### Beispiel 17

Zu 407 mg der in Beispiel 16 hergestellten Verbindung, 253 mg 4-(tert-Butoxy)-benzoesäure und 394 g Triphenylphosphin in 8 ml Tetrahydrofuran wurden unter Rühren 262 mg Diethylazadicarboxylat in 2 ml Tetrahydrofuran gegeben. Nach 4 Stunden Rühren bei 50°C wurde das Lösungsmittel unter vermindertem Druck entfernt und der Rückstand in 20 ml Cyclohexan aufgenommen, einmal mit 20 ml und zweimal mit je 10 ml 70% Methanol/Wasser gewaschen. Die wässrig-alkoholische Phase wurde zweimal mit je 10 ml Cyclohexan extrahiert. Die vereinigten Cyclohexanphasen wurden über Natriumsulfat getrocknet und das Lösungsmittel unter vermindertem Druck entfernt. Das zurückbleibende viskose Oel wurde in 10 ml heissem Heptan gelöst, mit reinem Endprodukt beimpft und 18 Stunden bei Raumtemperatur kristallisieren gelassen. Man erhielt 241 mg (3R,4R)-3-(4-tert-Butoxy-benzoylamino)-4-(4-tert-butoxy-benzoyloxy)-azepan-1-carbonsäure- tert-butylester vom Schmelzpunkt 126-128°C. Diese Verbindung kann wie in Beispiel 7b weiter umgesetzt werden.

### Beispiel 18

Zu 1,94 g 4-(tert-Butoxy)-benzoesäure und 2,63 g 4-Dimethylaminopyridin in 20 ml Dichlormethan wurden bei Raumtemperatur 12,91 g p-Toluolsulfonylchlorid, gelöst in 15 ml Dichlormethan im Verlauf von 15 Minuten gegeben. Das Reaktionsgemisch wurde 2 Stunden gerührt und im Verlauf von 1 Stunde zu 2,30 g (3R,4R)-3-Amino-4-hydroxy-azepan-1-carbonsäure-tert-butylester, gelöst in 10 ml Dichlormethan gegeben. Nach 1 Stunde Rühren wurde das Reaktionsgemisch mit 20 ml 1N NaOH, 20 ml 1N HCl und 20 ml Wasser gewaschen. Alle wässrigen Phasen wurden nacheinander mit 10 ml Dichlormethan gewaschen. Die vereinigten organischen Phasen wurden über Natriumsulfat getrocknet, filtriert und das Lösungsmittel abgedampft. Der erhaltene schaumige Rückstand wurde in 80 ml heissem Heptan gelöst und bei Raumtemperatur über Nacht kristallisiert. Die Kristalle wurden mit 10 ml Heptan gewaschen und getrocknet. Man erhielt 3,23 g (3R,4R)-3-(4-tert-Butoxy-benzoylamino)-4-hydroxy-azepan-1-carbonsäure-tert-butylester, Smp. 134-135°C.

### Beispiel 19

Zu 679 mg 4-Benzoyl-benzoesäure und 764 mg 4-Dimethylaminopyridin in 5 ml Dichlormethan wurden bei Raumtemperatur 572 mg p-Toluolsulfonylchlorid in 3,5 ml Dichlormethan im Verlauf von 10 Minuten gegeben. Nach weiterem Rühren bei Raumtemperatur während 2 Stunden wurden 1016 mg (3R,4R)-3-(4-tert-Butoxy-benzoylamino)-4-hydroxy-azepan-1-carbonsäure-tert-butylester in 2,5 ml Dichlormethan im Verlauf von 10 Minuten unter Rühren gegeben. Danach wurde das Reaktionsgemisch weitere 2,5 Stunden bei Raumtemperatur gerührt, mit 6 ml 1N NaOH, 6 ml 1N HCl und 6 ml Wasser gewaschen. Alle wässrigen Phasen wurden nacheinander mit 6 ml Dichlormethan extrahiert. Die vereinigten organischen Phasen wurden über Natriumsulfat getrocknet und das Lösungsmittel abgedampft. Das Rohprodukt wurde über 100 g Silicagel mit 1,4 l Hexan/Ethylacetat (2:1) chromatographiert. 100 ml-Fraktionen wurden gesammelt. Die Fraktionen 5-9 wurden vereinigt und das Lösungsmittel abgedampft. Man erhielt 1,48 g eines weissen Schaums, der aus 50 ml heissem Heptan kristallisiert wurde. Man erhielt 1,24 g (3R,4R)-3-(4-tert-Butoxy-benzoylamino)-4-(4-benzoyl-benzoyloxy)-azepan-1-carbonsäure-tert- butylester, Smp. 145-148°C als weisses Pulver.

### Beispiel 20

Zu 922 mg des in Beispiel 19 hergestellten Azepins in 4,0 ml Ethylacetat wurden bei Raumtemperatur unter Rühren 3,0 ml 5N HCl in Ethylacetat gegeben. Das Reaktionsgemisch wurde über Nacht bei Raumtemperatur gerührt, der Niederschlag abfiltriert, dreimal mit 2 ml Ethylacetat gewaschen und 16 Stunden bei 50°C/25 mbar getrocknet. Man erhielt 0,70 g 3-(4-Hydroxy-benzoylamino)-4-(4-benzoyl-benzoyloxy)-hexahydroazepinhydrochlorid.

## Patentansprüche

1. Verfahren zur Herstellung von Verbindungen der Formel I 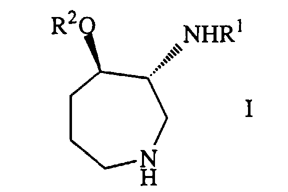 worin R¹ und R² unabhängig voneinander den Acylrest einer aromatischen Carbonsäure bedeuten,
dadurch gekennzeichnet, dass man
a) eine Verbindung der Formel II 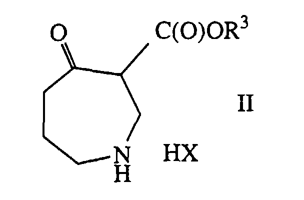 worin R³ nieder-Alkyl und HX eine Säure bedeutet,
a1) zu einer Verbindung der Formel IV 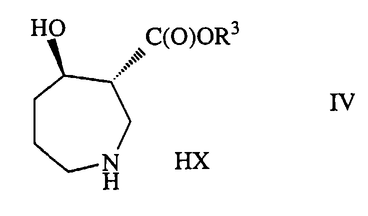 hydriert,
a2) die Verbindung der Formel IV in eine Verbindung der Formel V 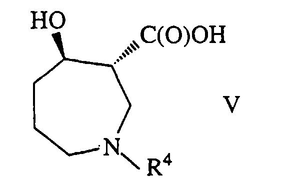 wobei R⁴ eine Schutzgruppe bedeutet,
umwandelt,
a3) die Verbindung der Formel V in eine Verbindung der Formel VI 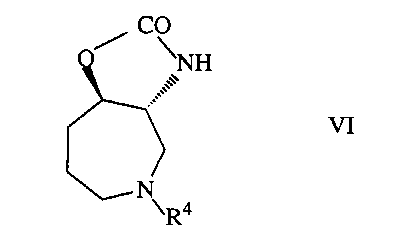 überführt,
a4) die Verbindung der Formel VI in eine Verbindung der Formel VII 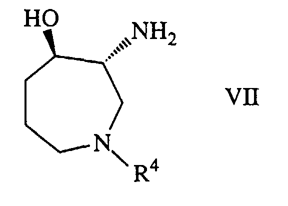 überführt,
a5) die Verbindung der Formel VII mit einer aromatischen Carbonsäure der Formel R¹COOH und/oder R²COOH N- bzw. O-acyliert;
oder dass man
b) eine Verbindung der Formel III 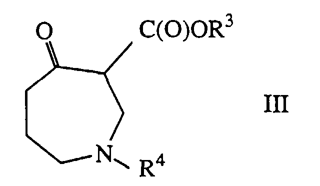 worin R³ und R⁴ die obengenannte Bedeutung haben,
b1) mikrobiell zu einer Verbindung der Formel VIII 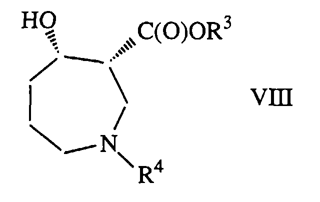 reduziert,
b2) die Verbindung der Formel VIII zu einer Verbindung der Formel VIII a verseift in diese in eine Verbindung der Formel IX 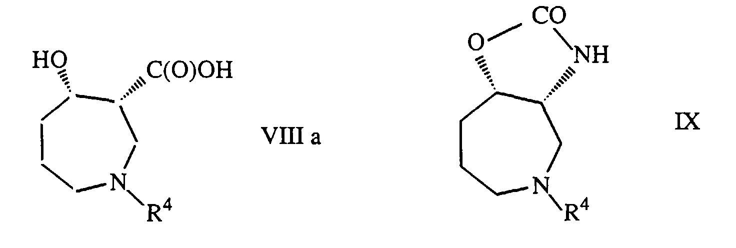 umwandelt,
b3) die Verbindung der Formel IX in eine Verbindung der Formel X 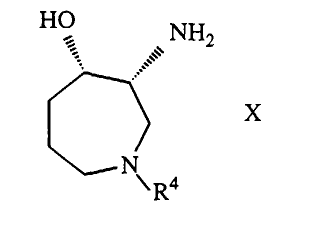 überführt,
b4) die Verbindung der Formel X mit einer aromatischen Carbonsäure der Formel R¹COOH zu einer Verbindung der Formel XI 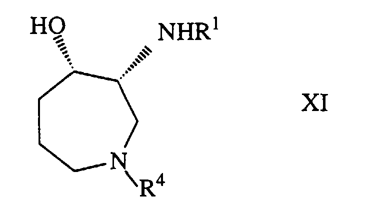 acyliert,
b5) die Verbindung der Formel XI mit einer aromatischen Carbonsäure oder einem reaktionsfähigen Derivat davon acyliert,
und aus der gemäss a5) oder b5) erhaltenen Verbindung der Formel XII 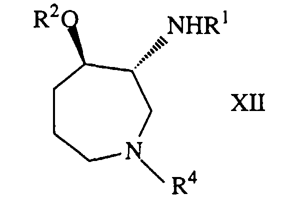 die Schutzgruppe R⁴ und gegebenenfalls in R¹ und/oder R² zusätzlich vorhandene Schutzgruppen abspaltet.

2. Verfahren nach Anspruch 1, wobei die Verbindung der Formel II in Gegenwart einer Verbindung der Formeln
[RuL]²⁺(X)₂ III-a
[RuLX²]²⁺(X)₂ III-b
[RuLX¹X²]⁺X³ III-c
RuL(X⁴)₂ III-d
worin
X BF₄⁻, ClO₄⁻, B(Phenyl)₄⁻, SbF₆⁻, PF₆⁻, Z¹-SO₃⁻,
X¹ Halogenid,
X² Benzol, Hexamethylbenzol oder p-Cymol,
X³ Halogenid, ClO₄⁻, B(Phenyl)₄⁻, SbF₆⁻, PF₆⁻, Z¹-SO₃⁻ oder BF₄⁻,
X⁴ ein Anion Z²-COO⁻ oder Z³-SO₃⁻, Allyl oder den Acetylacetonatrest (CH₃COCH=C(CH₃)O-);
Z¹ halogeniertes niederes Alkyl oder halogeniertes Phenyl,
Z² niederes Alkyl, Phenyl, halogeniertes niederes Alkyl oder halogeniertes Phenyl,
Z³ niederes Alkyl oder Phenyl bedeuten, und
L einen optisch aktiven, vorzugsweise atropisomeren Diphosphin-Liganden bedeuten,
als Katalysator hydriert wird.

3. Verfahren nach Anspruch 2, wobei L
MeOBIPHEP (6,6'-Dimethoxybiphenyl-2,2'-diyl)bis(diphenylphosphin)
BIPHEMP (6,6'-Dimethylbiphenyl-2,2'-diyl)bis(diphenylphosphin)
BINAP [(1,1'-Binaphthyl)-2,2'-diyl]bis(diphenylphosphin)
pTol-BIPHEMP (6,6'-Dimethylbiphenyl-2,2'-diyl)bis(di-(p-tolyl)phosphin)
pAn-MeOBIPHEP 6,6'-Dimethoxy-P,P,P',P'-tetrakis-(4-methoxyphenyl)-biphenyl-2,2'-bis-phosphin
pDMA-MeOBIPHEP 6,6'-Dimethoxy-P,P,P',P'-tetrakis-(4-dimethylamino-phenyl)-biphenyl-2,2'-bis-phosphin
pPhenyl-MeOBIPHEP (6,6'-Dimethoxybiphenyl-2,2'-diyl)bis[bis(biphenyl)-phosphin]
mTol-BIPHEMP (6,6'-Dimethylbiphenyl-2,2'-diyl)bis(di-(m-tolyl)phosphin)
Cy₂-MeOBIPHEP P2,P2-Dicyclohexyl-6,6'-dimethoxy-P2',P2'-diphenyl-biphenyl-2,2'-bis-phosphin
2-Furyl₂-BIPHEMP P,P-Diphenyl-P',P'-di-2-furyl-(6,6'-dimethylbiphenyl-2,2'-diyl)diphosphin
(3,5-Me,4-MeO)-MeOBIPHEP 6,6'-Dimethoxy-P,P,P',P'-tetrakis-(dimethyl-4-methoxy-phenyl)-biphenyl-2,2'-bis-phosphin
DiMeOBIPHEP (5,5',6,6'-Tetramethoxybiphenyl-2,2'-diyl)bis(diphenylphosphin)
TriMeOBIPHEP (4,4',5,5',6,6'-Hexamethoxybiphenyl-2,2'-diyl)bis(diphenylphosphin) oder
2-Furyl-MeOBIPHEP (6,6'-Dimethoxybiphenyl-2,2'-diyl)bis(di-2-furylphosphin)
ist.

4. Verfahren nach Anspruch 1, wobei die Verbindung der Formel II in Gegenwart von Ru(OAc)₂(R)-MeOBIPHEP als Katalysator hydriert wird.

5. Verfahren nach Anspruch 1, wobei die Verbindung der Formel III mittels einer Kultur von Hanseniaspora uvarum R 1052 reduziert wird.

6. Verfahren nach den Ansprüchen 1-5, wobei R¹ und R² p-Hydroxybenzoyl bedeuten.

7. Die Verbindungen der Formel IV gemäss Definition in Anspruch 1.

8. (3R,4R)-4-Hydroxy-azepan-carbonsäure-ethylester hydrochlorid.

9. Die Verbindungen der Formel V gemäss Definition in Anspruch 1.

10. (3R,4R)-4-Hydroxy-azepan-1,3-dicarbonsäure-1-tert.-butylester.

11. Verbindungen der Formel Va 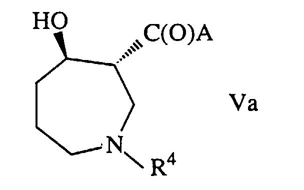 worin A Azido oder Amino und R⁴ eine Schutzgruppe bedeutet.

12. Die Verbindungen der Formel VI gemäss Definition in Anspruch 1.

13. (3aR,8aR)-5-tert-Butoxycarbonyl-2-oxo-octahydro-oxazolo[4,b-c]azepin.

14. Die Verbindungen der Formel VIII gemäss Definition in Anspruch 1.

15. (3R,4S)-1-(tert-Butoxycarbonyl)-4-hydroxy-azepan-3-carbonsäureethylester.

16. Die Verbindungen der Formel VIIIa gemäss Definition in Anspruch 1.

17. (3R,4S)-4-Hydroxy-azepan-1,3-dicarbonsäure-1-tert-butylester.

18. Die Verbindungen der Formel IX gemäss Definition in Anspruch 1.

19. (3aR,8aS)-2-Oxo-octahydro-oxazolo[4,b-c]azepin-5-carbonsäure-tert-butylester.

20. Die Verbindungen der Formel X gemäss Definition in Anspruch 1.

21. (3R,4S)-3-Amino-4-hydroxy-azepan-1-carbonsäure-tert-butylester.

22. Die Verbindungen der Formel XI gemäss Definition in Anspruch 1.

23. (3R,4S)-3-(4-tert-Butoxy-benzoylamino)-4-hydroxy-azepan-1-carbonsäure-tert-butylester.

24. (3R,4R)-3-(4-tert-Butoxy-benzoylamino)-4-(4-tert-butoxy-benzoyloxy)azepan-1-carbonsäure-tert-butylester.
